# FASCICULE DE BREVET EUROPEEN

(11) **EP 3 600 559 B1**
(45) Date de publication et mention de la délivrance du brevet: **13.10.2021**
(21) Numéro de dépôt: 18718590.5
(22) Date de dépôt: 26.03.2018
(51) Int. Cl.: A61Q 1/02, A61Q 1/08, A61Q 1/12, A61Q 17/04, A61K 8/06, A61K 8/87, A61K 8/90, A45D 33/00, A45D 40/22

(54) **ARTICLE COSMÉTIQUE MUNI D'UNE MAILLE**
KOSMETIKARTIKEL MIT EINEM NETZ
COSMETIC ARTICLE PROVIDED WITH A MESH

(30) Priorité: 31.03.2017 FR 1752768
(43) Date de publication de la demande: 05.02.2020
(73) Titulaire: Chanel Parfums Beauté, 92200 Neuilly-sur-Seine (FR)
(72) Inventeur: VILLECROZE, Hélène, 93694 Pantin Cedex (FR); LAUER, Alexandre, 93694 Pantin Cedex (FR); MILESI, Frédéric, 92200 Neuilly sur Seine Cedex (FR); MENARD, Caroline, 60300 Chamant (FR)
(74) Mandataire: Plasseraud IP
(86) Numéro de dépôt international: PCT/FR2018/050726
(87) Numéro de publication internationale: WO 2018/178550

(56) Documents cités:
- EP-A1- 1 588 644
- EP-A1- 2 837 306
- EP-A1- 3 058 843
- DE-A1- 19 929 900
- FR-A1- 2 792 180
- FR-A1- 2 838 618
- FR-A1- 2 949 959
- US-A1- 2017 079 408

## Description

La présente invention concerne le domaine de la cosmétique, en particulier les domaines du maquillage, du soin, ou des compositions parfumantes.

Bien qu'elle n'y soit pas cantonnée, l'invention a des applications particulièrement intéressantes dans le domaine plus spécifique des compositions pour le visage, telles que le fond de teint, le correcteur de teint, la base de teint, l'anti-cernes, le fard à joues ― parfois appelé *blush,* l'illuminateur ― parfois appelé *highlighter,* les soins hydratants, les soins anti-rides, et les soins de protection solaire.

Les produits cosmétiques, en particulier les produits de maquillage, sont traditionnellement des produits à utiliser chez soi. Avec l'évolution des modes de vie, de plus en plus de consommateurs les transportent pour une utilisation en extérieur, mais les produits ne sont pas toujours adaptés à un tel usage. Les produits cosmétiques outdoor ― ou nomades ― doivent être compacts et ne pas être trop encombrant pour permettre une utilisation facile et optimale du produit en toutes circonstances, de la même manière que dans le confort d'une salle de bain.

Parmi les produits cosmétiques compacts existants, les poudres compactées et les produits coulés sont les plus courants. Cependant, de telles compositions ne conviennent pas à tous les types de peaux ni à toutes les applications. Notamment, les poudres compactes ne sont pas compatibles avec des compositions de soin, et les produits coulés ne présentent généralement pas de bonnes propriétés d'étalement du fait de l'utilisation de cires structurantes.

Les compositions cosmétiques fluides en revanche sont très appréciées pour leurs propriétés d'étalement sur la peau. Elles procurent de plus grandes satisfactions au consommateur, notamment en termes de résultat maquillage dans le cas d'un produit de maquillage, de sensorialité, de confort, de fraicheur et/ou de tenue.

Parmi les articles de conditionnement compacts pour des compositions cosmétiques fluides, il existe notamment des éponges imprégnées. Ces produits, connus sous le nom de *cushion,* constituent une nouvelle catégorie de produits très appréciée des consommateurs pour leur nomadisme. Une éponge poreuse, par exemple de type urethane, est placée dans un boitier compact, et est imprégnée d'une composition cosmétique fluide, comme le décrit le document WO2012128589. Le produit est alors prélevé en pressant l'éponge imprégnée à l'aide d'un applicateur, par exemple une seconde éponge non poreuse. Cependant, la dose prélevée peut être très variable selon la pression exercée par l'utilisateur, et peut même conduire à un débordement de la composition fluide en dehors de l'éponge. Ce type de mise en œuvre présente également de nombreuses contraintes techniques. Il nécessite de maitriser parfaitement les paramètres structurels de l'éponge, notamment sa porosité et sa dureté. De plus, un tel article cosmétique ne permet pas une restitution complète du produit au consommateur du fait de l'absorption et de l'adsorption du produit par l'éponge. Ainsi, pour compenser la perte de produit, il est nécessaire d'augmenter la taille de l'éponge pour augmenter la quantité de produit, ce qui ne favorise pas la portabilité du dispositif.

On connait par ailleurs des dispositifs comprenant un filet tendu au-dessus d'un réservoir, retenant une composition fluide à crémeuse, comme décrit dans le document KR101477583. Dans ce cas, le filet doit être suffisamment perméable pour laisser passer la composition cosmétique, mais sans l'être de façon excessive car la composition pourrait s'écouler à travers le filet qui ne remplirait plus sa fonction de rétention de la composition. Ce document décrit notamment une fourchette de viscosité optimale comprise entre 20 et 40 Pa.s pour éviter les problèmes de fuite. En dehors de cette gamme de viscosité, le dispositif décrit n'est plus adapté. Or, les compositions cosmétiques présentant de bonnes propriétés d'étalement se situent idéalement en dessous de 10 Pa.s, de préférence en dessous de 7 Pa.s, plus préférentiellement entre 1 Pa.s et 3 Pa.s. En outre, le filet de l'article cosmétique de ce document s'intercale entre deux guides circulaires venant s'emboîter l'un dans l'autre grâce à des lèvres et des saillies, cet ensemble venant ensuite reposer sur les parois du corps du réservoir. Deux autres éléments de fermeture sont nécessaires pour assurer l'étanchéité. Ce réservoir est composé de beaucoup de pièces qui n'assurent pas une étanchéité maximum. Le dispositif de KR101477583 n'est donc pas adapté au conditionnement et à une utilisation nomade des compositions cosmétiques présentant de bonnes propriétés d'étalement.

Le document EP2837306 décrit un dispositif combinant les différentes approches susmentionnées : une maille est associée à un matériau absorbant pour délivrer le produit cosmétique. Le matériau absorbant est typiquement une éponge et sert de conteneur à une composition cosmétique liquide. Il permet aussi d'éjecter ce même liquide à travers le tamis lorsqu'une force est verticalement appliquée. Ce type de produit comporte les inconvénients des éponges sans les avantages de l'utilisation d'une maille, à savoir une délivrance homogène, un conditionnement faible et une consommation complète du produit.

Un but de l'invention est donc de fournir un article cosmétique destiné à un usage nomade permettant le conditionnement et l'utilisation d'une composition cosmétique présentant des propriétés optimales, et notamment un bon étalement. Un tel article cosmétique doit notamment être compact et prémunir l'utilisateur de tout risque de fuite ou de débordement lors du transport du dispositif et de son utilisation.

A cet effet, on prévoit selon l'invention un article cosmétique comprenant :
i. Un boitier,
ii. Un réservoir logé dans le boîtier, contenant une composition cosmétique thixotrope, et présentant une ouverture pour délivrer ladite composition cosmétique,
iii. Une maille recouvrant ladite ouverture du réservoir, configurée pour être imperméable à la composition cosmétique lorsque celle-ci n'est pas soumise à une contrainte de cisaillement, et perméable à la composition cosmétique lorsque celle-ci est soumise à une contrainte de cisaillement.

L'utilisation d'une composition cosmétique thixotrope en combinaison avec une maille permet de combiner une composition cosmétique présentant d'excellentes propriétés d'étalement à un usage nomade. En effet, lors de son prélèvement et de son étalement, par exemple à l'aide d'une éponge, la composition est soumise à une contrainte de cisaillement, ce qui la déstructure et la rend fluide, un bon étalement s'en suivant. La composition cosmétique peut ainsi procurer de la fraicheur et de l'hydratation. En outre, cela permet un prélèvement facilité de la composition à travers la maille : le consommateur peut contrôler précisément la dose de composition prélevée.

Avant utilisation et une fois l'utilisation terminée, la composition cosmétique se restructure et présente une viscosité suffisamment importante pour ne pas pouvoir traverser la maille. Cela permet une utilisation nomade de l'article cosmétique. En effet, la composition cosmétique ne peut pas fuir de l'article cosmétique dans son état natif, qui est un état structuré et visqueux. La composition cosmétique n'est pas non plus susceptible de déborder puisque la maille est imperméable à la composition cosmétique lorsque celle-ci n'est pas soumise à une contrainte de cisaillement, c'est-à-dire lorsqu'elle est dans son état natif.

Le comportement rhéologique de la composition cosmétique permet ainsi une grande compatibilité avec les boitiers nomades munis d'une maille, tout en s'affranchissant des contraintes de l'art antérieur précédemment décrit. L'article cosmétique selon l'invention peut être utilisé sans éponge imprégnée, ce qui permet de réduire considérablement le volume du réservoir, et de prévenir toute perte de produit. L'absence du risque d'écoulement lors du transport de la composition réduit fortement les contraintes de conception du packaging de l'article cosmétique, notamment de la maille.

Le terme maille au sens de l'invention désigne une alternance de fibres organisées en réseau. Il peut typiquement s'agir d'un tissu, d'un filet ou encore d'un grillage métallique. Les fibres peuvent être de tout type, notamment textile ou métallique, mais sont de préférence en un matériau polymère. Les fibres peuvent être toutes identiques, ou au contraire être différentes. De préférence, la maille comprend des fibres en polyéthylène téréphtalate (PET) et des fibres en polyuréthane. Une proportion importante de fibres en polyuréthane peut avantageusement être utilisée afin de conférer à la maille une élasticité importante.

Avantageusement, la maille est suffisamment élastiquement déformable pour permettre d'atteindre le fond du réservoir. Cela permet à la composition cosmétique d'être totalement restituable au consommateur, sans perte de produit due au moyen de conditionnement.

L'organisation des fibres en réseau peut être de n'importe quel type, mais de préférence, elles sont tissées en tricot. Un tissage en tricot permet de donner une bonne rigidité à la maille tout en tirant parti de l'élasticité des fibres élastiques que contient éventuellement la maille. L'organisation des fibres de la maille définit des espacements entre les fibres. On appellera *pas* la dimension moyenne de ces espacements. Le pas de la maille et les dimensions des plus grands espacements entre les fibres impacteront fortement la perméabilité de la maille. De préférence, le pas de maille est compris entre 0,01 et 1mm.

La maille de l'article cosmétique selon l'invention est configurée pour être imperméable à la composition cosmétique lorsque celle-ci n'est pas soumise à une contrainte de cisaillement, et perméable à la composition cosmétique lorsque celle-ci est soumise à une contrainte de cisaillement. Afin de configurer la maille de la sorte, on pourra faire varier le pas de la maille, la dimension des plus grands de ces espacements, ou encore le matériau dans lequel sont faites les fibres de la maille. En effet, une maille faite de fibres hydrophobes sera par exemple moins perméable à des compositions hydrophiles, et vice versa. Ces ajustements ne présenteront pas de difficulté particulière pour l'homme de l'art. Des cas particuliers seront illustrés dans les exemples de réalisation de l'invention, détaillés plus bas.

La composition cosmétique contenue dans l'article cosmétique selon l'invention comprend de préférence une émulsion huile-dans-eau ayant une phase aqueuse et une phase grasse, la phase aqueuse comprenant un agent gélifiant thixotrope. L'utilisation d'émulsions huile-dans-eau permet d'obtenir une formule très fraiche à l'application, ce qui est très apprécié des consommateurs

L'agent gélifiant thixotrope est de préférence un copolymère séquencé non ionique comportant au moins une portion hydrophile et une portion hydrophobe. Les copolymères séquencés sont aussi connus sous le nom de polymères associatifs ou polymères à blocs.

Du fait de la présence conjointe de portions hydrophobes et hydrophiles, et bien que dans le mode de réalisation préféré de l'invention l'agent thixotrope soit compris dans la phase aqueuse d'une émulsion huile-dans-eau, celui-ci pourrait également être compris dans la phase grasse de l'émulsion ou même à l'interface entre les phases, par exemple au sein d'un éventuel agent émulsifiant, sans sortir du champ de la présente invention. Toutefois, le fait que l'agent thixotrope soit compris dans la phase aqueuse permet aux différentes macromolécules d'interagir de façon réversible via leurs portions hydrophobes, ce qui favorise la formation d'un gel aux propriétés thixotropes.

Les portions hydrophiles et les portions hydrophobes de cet agent thixotrope sont par exemple connectées par au moins une liaison uréthane.

De même, le copolymère séquencé non ionique pourra avantageusement être choisi parmi les polyuréthanes et leurs dérivés.

Les liaisons uréthane présentent de nombreux avantages, notamment celui d'être compatible avec une utilisation cosmétique. Les groupes uréthane présentent en outre une excellente stabilité et une rigidité exceptionnelle ce qui se traduit par de très bonnes propriétés mécaniques de l'agent thixotrope, y compris lorsque les groupes uréthane ne sont présents qu'à la jonction entre les portions hydrophiles et les portions hydrophobes de l'agent thixotrope.

Dans un mode de réalisation avantageux, la portion hydrophile d'un tel agent thixotrope est polyoxyéthylénée ― il peut par exemple s'agir de polyéthylène glycol (PEG) et/ou la portion hydrophobe est hydrocarbonée, par exemple des chaines hydrocarbonées comprenant entre 6 et 40 atomes de carbone. Les polymères polyoxyéthylènés, et notamment le PEG, ont généralement une viscosité qui diminue lorsque la température augmente. Ces polymères très hydrophiles présentent en outre une bonne stabilité à la chaleur et au dioxygène, ce qui est particulièrement avantageux pour une utilisation cosmétique nomade. En effet, l'article cosmétique sera plus régulièrement exposé à la chaleur dans le cas d'un usage nomade que dans le cas d'une utilisation dans une salle de bain. En particulier, l'article cosmétique peut être placé dans la poche d'un vêtement et réchauffé par la proximité du corps de l'utilisateur. Il peut également être laissé en plein soleil par inadvertance.

De préférence, le copolymère séquencé non ionique est constitué d'une chaîne de trois blocs successifs, le premier et le troisième bloc présentant des propriétés hydrophiles, et le deuxième bloc présentant des propriétés hydrophobes. Par exemple, le copolymère peut présenter une structure de type A-B-A′ dans laquelle A et A′ représentent les portions hydrophobes, et B la portion hydrophile. Les portions A et A′ peuvent être identiques ou différentes. Les portions hydrophobes à chaque extrémité peuvent alors s'assembler, de façon réversible, et forment ainsi un réseau tridimensionnel responsable de la gélification de la composition, et de son caractère thixotrope. Un tel copolymère est par exemple le PEG-240/HDI COPOLYMER BIS-DECYLTETRADECETH-20 ETHER (nom INCI), et disponible sous la référence Adeka Nol GT-700, ou Adeka Nol GT-730 de la société Adeka. Ce polymère est utilisé comme agent thixotrope dans un mode de réalisation préféré de l'invention.

La teneur en poids de l'agent gélifiant thixotrope par rapport au poids total de la composition est compris entre 0,01% et 20%, de préférence entre 0,5 et 10%, encore de préférence de 1 à 5%. La teneur doit être suffisante pour conférer à la composition les propriétés rhéologiques souhaitées, et pourra donc varier selon l'agent thixotrope choisi.

La composition cosmétique doit présenter un comportement rhéologique particulier, favorisant le conditionnement et la distribution de la composition par l'article cosmétique selon la présente invention, destiné à un usage nomade. En particulier, la composition a un caractère rhéofluidifiant : après l'application d'un cisaillement, la viscosité de la composition diminue. Par "composition cosmétique thixotrope" on entend une composition cosmétique ayant au moins le caractère rhéofluidifiant. De plus, après un temps de repos, la composition régénère partiellement ou totalement sa viscosité, sa consistance et son élasticité initiale. La reprise en viscosité, consistance, élasticité est différée dans le temps. La composition peut avantageusement présenter un comportement à mémoire de forme, c'est-à-dire qu'elle peut être déformée en une configuration temporaire, sous l'effet d'une contrainte mécanique par exemple, et ensuite être restaurée à la géométrie originale au bout d'un certain temps après l'arrêt de la contrainte. Une telle composition est parfois qualifiée de gel faible. Macroscopiquement, cela se traduit par le fait que lorsque qu'un utilisateur prélève de la composition dans un récipient contenant la composition, la surface redevient lisse et horizontale comme initialement après un certain temps. Cette propriété permet non seulement de garder le même aspect esthétique que lors de la première utilisation de l'article cosmétique selon la présente invention, mais elle permet également à l'utilisateur de contrôler la dose de composition prélevée à chaque nouvelle utilisation, et ce, jusqu'à épuisement total de la composition cosmétique. En effet, lorsque la composition se replace uniformément derrière la maille, cela permet à l'utilisateur de prélever à chaque fois la même dose.

La composition cosmétique présente de préférence une viscosité de plateau supérieure ou égale à 2500 Pa.s, de préférence supérieure ou égale à 2700 Pa.s.

La composition cosmétique présente de préférence un seuil d'écoulement inférieur ou égal à 60 Pa, de préférence inférieur ou égal à 55 Pa. Le seuil d'écoulement correspond à la contrainte minimale pour mettre en écoulement le produit. Cette valeur donne une information sur la facilité d'étalement de la composition et sa capacité à être extrudé à travers la maille. Un seuil d'écoulement suffisamment faible est nécessaire pour faciliter le prélèvement.

La composition cosmétique présente de préférence un module complexe G^{∗} compris entre 200 et 600 Pa, de préférence entre 300 et 500 Pa.

La composition présente de préférence un angle de phase δ compris entre 7° et 20°, de préférence entre 7° et 10°.

La composition présente de préférence une viscosité à 100 s⁻¹ inférieure à 3 Pa.s, de préférence comprise entre 1 et 3 Pa.s. La viscosité à une vitesse de cisaillement de 100 s⁻¹, correspond à une contrainte équivalente à celle exercée au moment de l'étalement sur la peau Ces différentes propriétés sont mesurées selon les protocoles suivants.

Les différentes viscosité de la composition contenue dans l'article selon l'invention est mesurée à 20°C à l'aide du rhéomètre DHR2 de la société TA Instruments, équipé d'un mobile Plan-Plan sablé de 40 mm de diamètre. Les mesures sont effectuées en suivant une rampe d'écoulement. La composition est d'abord mise en température à 20°C ± 1°C pendant 180 secondes sans qu'aucun cisaillement ne lui soit appliqué. Puis, un balayage en contrainte est appliqué à la composition pendant 400 secondes entre 0,01 Pa à 1000 Pa.

On représente graphiquement l'évolution de la viscosité η en fonction du gradient de cisaillement appliqué σ. Les points de mesure sont répartis de façon logarithmique, à raison de 50 points par décade. La viscosité de plateau correspond à la viscosité au repos. Graphiquement, il s'agit des premiers points formant un plateau aux faibles cisaillements.

L'état de structure des compositions est étudié via un balayage en oscillation en rampe de contrainte à l'aide du rhéomètre DHR-2 de la société TA Instruments. L'appareil est muni d'un mobile plan-plan "sablé" de 40 mm de diamètre. La composition est mise à température à 20°C pendant 60 secondes sans qu'aucune contrainte ne lui soit appliquée. Puis, un balayage en contraintes croissantes est appliqué entre 0,01 à 1000 Pa à une fréquence fixe de 1 Hz. On mesure ainsi l'évolution du module complexe G^{∗} (en Pa) en fonction de la contrainte en oscillation appliquée (en Pa).

Cette méthode permet de déterminer les paramètres suivants:
- le module de cisaillement complexe G^{∗}(en Pa) de la composition qui permet de caractériser la consistance de la composition, et ses propriétés viscoélastiques. Il correspond au rapport de la contrainte imposée sur la déformation mesurée.
- l'angle de phase δ (en degrés) qui donne une information sur le comportement plus ou moins solide ou liquide de la composition. L'angle de phase d'un produit est compris entre 0 et 90° (à 0° on parle de solide parfait, et à 90° de liquide parfait).

Les valeurs de G^{∗} et de δ retenues sont les valeurs "plateau", c'est-à-dire correspondant à la zone de contrainte pour laquelle il y a moins de 10% de variation de ces deux grandeurs.

La composition cosmétique contenue dans l'article cosmétique selon l'invention peut comprendre d'autres composants.

### Composition - phase aqueuse

La composition cosmétique comprend une ou plusieurs phases aqueuses. On entend par "phase aqueuse", une phase comprenant de l'eau et en général toute molécule à l'état dissous dans l'eau dans la composition.

La phase aqueuse des dites compositions contient de l'eau et en général d'autres solvants solubles ou miscibles dans l'eau. Les solvants solubles ou miscibles dans l'eau comprennent les mono alcools à chaîne courte par exemple en C1 -C4 comme l'éthanol, l'isopropanol; les agents humectant tels que les sels d'acide hyaluronique comme le sodium hyaluronate, les diols ou les polyols comme l'éthylèneglycol, le 1,2-propylèneglycol, le 1,3 - butylène glycol, le caprylyl glycol, l'hexylèneglycol, le diéthylèneglycol, le dipropylène glycol, et leurs mélanges. On peut citer également les dérivés de polysaccharides obtenus par fermentation bactérienne tel que le composé connu sous le nom INCI Biosaccharide gum-1.

La composition peut comprendre en outre un système émulsionnant, ou émulsifiant. Le système émulsionnant peut consister en un tensioactif ayant un HLB (balance hydrophile/lipophile) inférieur à 7. De préférence, le tensioactif émulsionnant est alors choisi parmi les ester d'acides gras de polyols comme les mono-, di-, tri- ou sesqui-oléates ou stéarates de sorbitol ou de glycérol, les laurates de glycérol ou de polyéthylène glycol les tensioactifs siliconés comme les diméthicones copolyols ou les alkyl dimethicone copolyol à chaîne alkyle pendante ou en bout de squelette siliconé ayant par exemple de 6 à 22 atomes de carbone, tels que le PEG-10 dimethicone; les polymères du type ester d'acide gras de glycol polyoxyalkyléné, les alkyl ou alkoxy diméthicones copolyols à chaîne alkyle ou alkoxy pendante ou en bout de squelette siliconé ayant par exemple de 6 à 22 atomes de carbone, et leurs mélanges.

La composition peut également comprendre au moins un gélifiant de la phase aqueuse différent du gélifiant cité précédemment destiné à conférer les propriétés rhéologiques précitées. Les principaux agents gélifiants hydrophiles utilisables sont les gélifiants polymériques réticulés et les polymères naturels.

De tels gélifiants polymériques réticulés sont par exemple les polymères carboxyvinyliques, comme les produits commercialisés sous les dénominations Carbopol (nom INCl : carbomer) par la société Novéon, les polyacrylamides, les polymères dérivés de l'acide 2-acrylamido-2-méthylpropane sulfonique (AMPS) tels que le produit commercialisé par la société Clariant sous la dénomination Hostacerin AMPS, et les copolymères anioniques réticulés d'acrylamide et d'AMPS, tels que le produit commercialisé sous le nom de SEPIGEL 305 ou SEPINOV EMT 10 par la société Seppic.

Les polymères naturels utilisables sont par exemple la gomme gellane, la gomme de xanthane, et la gomme de guar, ou encore les dérivés cellulosiques, les amidons et les alginates, et leurs mélanges. Ces polymères naturels peuvent être utilisés seuls ou en association avec les gélifiants polymériques réticulés.

### Composition - huiles

En outre, la composition comprend de préférence 0,01 à 60% en poids par rapport au poids total de la composition, plus préférentiellement entre 0,5 et 50%, encore plus préférentiellement entre 1 et 30% d'huiles.

Les huiles utilisables peuvent être choisies de préférence parmi des huiles hydrocarbonées, des huiles siliconées, ou leurs mélanges.

Au sens de la présente invention, on entend par « huile » un composé liquide à température ambiante (25°C) et pression atmosphérique, et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau. Les huiles utilisables dans la présente invention peuvent être volatiles ou non-volatiles. Les huiles peuvent être d'origine végétale, minérale ou synthétique.

Les huiles non volatiles d'origine végétale comprennent notamment l'huile de ricin, l'huile d'amande douce, l'huile de tournesol, l'huile de son de riz, l'huile de noix de macadamia, l'huile d'olive, l'huile de germe de blé, l'huile d'arachide, l'huile d'argousier, l'huile de bourrache, l'huile d'onagre, l'huile de palme, l'huile de camélia, l'huile de gardénia, et leurs mélanges.

Les huiles non volatiles d'origine minérale comprennent notamment la paraffine liquide, l'isoparaffine.

Les huile non volatiles d'origine synthétique comprennent notamment les huiles hydrocarbonées telles que les (poly)ethers ou (poly)esters, en particulier les (poly)esters d'acides gras en C6 à C20 et d'alcools gras en C6 à C20 qui peuvent être avantageusement branchés tels que diisostéaryl malate, le dicaprylyl carbonate, le isononyl isononanoate; les huiles végétales ou leurs dérivés tels que l'huile de ricin hydrogénée; les acides gras branchés et/ou insaturés; les alcools gras branchés et/ou insaturés; les huiles de silicone telles que les polydimethylsiloxanes linéaires (nom INCI: Dimethicone) qui peuvent être optionnellement phénylées, les polydimethylsiloxanes cycliques; et leurs mélanges.

A titre d'exemple, la composition contenue dans l'article selon l'invention pourra typiquement contenir une ou plusieurs huile non volatile choisie parmi les huiles hydrocarbonées telles que le squalane, le polybutene, le polyisobutene hydrogéné, ou le polydecene hydrogéné ; les huiles siliconées phénylées telles que celles identifiables par les noms INCI "phenyl trimethicone", phenylpropyldimethylsiloxysilicate" ou "trimethyl pentaphenyl trisiloxane" ; et les huiles de silicone fluorées.

La composition contenue dans l'article cosmétique selon l'invention peut également comprendre au moins une huile volatile à température ambiante. Par "huile volatile", on entend au sens de l'invention une huile susceptible de s'évaporer au contact de la peau ou de la fibre kératinique en moins d'une heure. Les huiles volatiles de l'invention sont des huiles cosmétiques volatiles, liquides à température ambiante, ayant une pression de vapeur non nulle, à température ambiante et pression atmosphérique. Les huiles volatiles utilisables dans le cadre de l'invention peuvent être siliconées ou hydrocarbonées.

Des exemples d'huiles volatiles hydrocarbonées appropriées comprennent des alcanes linéaires volatils tels que ceux décrits dans le document FR2933865. Des exemples d'alcanes linéaires volatils appropriés sont les alcanes en C9-C17, en particulier C10-C14, tels que le mélange d'undécane et de tridécane, disponible sous la dénomination commerciale Cetiol® Ultimate de BASF ou les alcanes en C15-C19 disponibles sous la dénomination commerciale Emogreen ® L15 de SEPPIC, ou alcanes en C12-14 disponibles sous le nom commercial Vegelight® 1214 LC de Biosynthis.

Des exemples d'huiles volatiles siliconées appropriées comprennent les huiles de silicone linéaires, ramifiées ou cycliques, en particulier présentant une viscosité inférieure à 10 cst, et en particulier ayant entre 2 et 10 atomes de silicium. Des exemples particuliers non limitatifs incluent l'octamethyl cyclotetrasiloxane, le decamethyl cyclopentasiloxane, le dodecamethyl cyclohexasiloxane, l'heptamethyl hexyltrisiloxane, l'heptamethyloctyl trisiloxane, hexamethyl disiloxane, octamethyl trisiloxane, decamethyl tetrasiloxane, dodecamethyl pentasiloxane, les composés dont les noms INCI correspondent à Methyl Trimethicone, et leurs mélanges.

Bien entendu la composition contenue dans l'article selon l'invention peut comprendre des mélanges des huiles mentionnées ci-dessus.

### Composition - filmogènes

La composition contenue dans l'article selon l'invention peut également comprendre au moins un composant filmogène, en particulier susceptible d'apporter de la tenue et/ou des propriétés de non -transfert à la composition.

Le filmogène est généralement un polymère. Ce polymère filmogène peut être un polymère siliconé éventuellement modifié uréthane ou fluoré ou acrylate tel que les silicones (méth)acrylates commercialisées par JEEN sous la dénomination Jéesil PS (qui inclus PS-VH, PS-VHLV, PS-CM, PS- CMLV et PS-DMLV),ou les polymères commercialisés par SHIN-ETSU sous les dénominations commerciales KP-545, KP-561 et KP-562, ou les polymères commercialisés par la société DOW CORNING sous les dénominations commerciales Dow Corning® FA 4003 DM, Dow Corning® FA 4002 ID et Dow Corning® FA 4001 CM. D'autres exemples de polymères filmogènes sont les résines de silicone et en particulier les résines MQ telles que les triméthylsiloxysilicates commercialisées par la société Wacker sous le nom Belsil TMS 803, et les résines MT telles que les dérivés de silsesquioxane et notamment les polyméthylsilsesquioxanes, commercialisés notamment par la société SHIN-ETSU, ainsi que le polypropylsilsesquioxane commercialisé par la société DOW CORNING sous la dénomination commerciale Dow Corning® 670 ou le phénylpropyl polysilsesquioxane commercialisé par la société WACKER sous la dénomination commerciale BELSIL SPR45VP. Un autre exemple est constitué des polymères fluorosiliconés identifiés par le nom INCI trifluoropropyldiméthylsiloxy triéthylsiloxysilicate tels que celui commercialisé par la société GENERAL ELECTRIC sous la dénomination commerciale XS66-B8226. On peut également utiliser comme polymères filmogènes des polymères bioadhésifs obtenus par exemple par polycondensation de diméthiconol et de résine silicate MQ dans un solvant tel que l'heptane, qui sont notamment commercialisés par la société DOW CORNING sous les dénominations commerciales Dow Corning® BIO-PSA 7-4560 SILICONE ADHESIVE, Dow Corning® 7-4405 low tack et Dow Corning® 7-4505 high tack. D'autres exemples de polymères filmogènes sont les polyoléfines cycliques telles que le polycyclopentadiène, notamment commercialisé par la société KOBO sous la dénomination commerciale KOBOGUARD 5400, ou encore le polydicyclopentadiène. D'autres exemples encore de filmogènes sont constitués de copolymères de vinylpyrrolidone (VP) et/ou d'oléfines linéaires tels que les copolymères VP/hexadécène et VP/eicosène dont l'ANTARON V216 et l'ANTARON V220 de la société ISP ou encore les copolymères éthylène / acétate de vinyle tels que l'AC 400 de la société BAERLOCHER. D'autres polymères filmogènes susceptibles d'être utilisés dans cette invention sont des polyacrylates tels que le poly(acrylate d'éthyle) commercialisé notamment par la société CREATIONS COULEURS sous la dénomination commerciale CREASIL 7 ID.

### Composition -filtres ultraviolets

La composition cosmétique peut également comprendre 0 à 50 % en poids d'au moins un filtre ultraviolet (UV), de préférence de 0 à 30%, plus préférentiellement de 0 à 20% en poids total de la composition. En particulier, la composition contenue dans l'article selon l'invention peut comprendre au moins un filtre solaire organique ou minéral ou un mélange des deux.

A titre d'illustration des filtres UV organiques et de façon non limitative, on peut citer:
- les anthranilates, en particulier l'anthranilate de menthyle ;
- les benzophénones, en particulier la benzophénone-1, la benzophénone-3 ou oxybenzone, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40® disponible chez B.A.S.F.) ;
- les benzylidènes-camphres, en particulier le 3-benzylidène-camphre, l'acide benzylidènecampho-sulfonique, le benzalkoniumméthosulfate de Camphre, le polyacrylamidométhylbenzylidène camphre, l'acide téréphthalylidène di-camphre sulfonique, et préférentiellement le 4-méthylbenzylidène camphre (Eusolex 6300® disponible chez Merck) ;
- les benzimidazoles, en particulier le benzimidazilate (Neo Heliopan AP® disponible chez Haarmann et Reimer), ou l'acide phénylbenzimidazole sulfonique (PARSOL HS® disponible chez DSM) ;
- les benzotriazoles, en particulier le drométrizole trisiloxane, ou le méthylène bis-benzotriazolyltétraméthylbutylphénol (Tinosorb M® disponible chez Ciba) ;
- les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, Kaempferia galanga root extract (TEGO GALANGA d'EVONIK contenant 98% ethyl-p-methoxycinnamate) et préférentiellement l'octylméthoxycinnamate (Parsol MCX® disponible chez Hoffmann La Roche) ;
- les diphenylacrylates en particulier l'éthocrylène (Uvinul N35® disponible chez B.A.S.F.), ou l'octocrylène (Uvinul 539® disponible chez B.A.S.F.) ou Ethylhexyl methoxycrylene (SOLASTAY disponible chez HALLSTAR) ;
- les dibenzoylméthanes, en particulier le butyl méthoxydibenzoylméthane (Parsol 1789®) ; les imidazolines, en particulier l'éthylhexyl diméthoxybenzylidène dioxoimidazoline;
- les PABA, en particulier l'éthyl Dihydroxypropyl PABA, l'éthylhexyldiméthyl PABA, le glycéryl PABA, le PABA, le PEG-25 PABA, ou l'éthyl PABA (benzocaïne) ;
- les triazines, en particulier l'anisotriazine (Tinosorb S® disponible chez Ciba) ou la diéthylhexylbutamido-triazone (Uvasorb HEB® disponible chez 3V Sigma), l'éthylhexyltriazone (Uvinul T150® disponible chez B.A.S.F.), le Tris-Biphenyl Triazine (Tinosorb 2AB disponible chez BASF) ;
- les benzoates, en particulier le N-hexyl 2-(4-diethylamino-2-hydroxybenzoyl)benzoate (Uvinul A+ disponible chez BASF) ou en mélange avec octyl methoxycinnamate (Uvinul A+B disponible chez BASF) ;
- les benzalmalonates, en particulier le Polysilicone-15 (Parsol SLX disponible chez DSM) :
- les benzoxazoles en particulier le 2,4-Bis[4-[5-(1,1 -dimethylpropyl)benzoxazol-2-yl]phenylimino]-6-[(2-ethylhexyl)imino]- 1,3,5-triazine, (Uvasorb K2A disponible chez Sigma 3V) ;
- les salicylates, en particulier le salicyclate de dipropylèneglycol, le salicylate d'éthylhexyle, l'homosalate, le butyloctyl Salicylate (HALLBRITE BHB disponible chez HALLSTAR ou le TEA salicylate.

Les filtres UV inorganiques utilisés sont par exemple des particules d'oxyde métallique ayant une taille moyenne de particule élémentaire inférieure ou égale à 300 nm, de préférence inférieure ou égale à 100 nm.

Ils peuvent être notamment choisis parmi les oxydes de titane, de zinc, de fer, de zirconium, de cérium ou leurs mélanges. Les oxydes de titane peuvent se présenter sous une forme cristallisée de type rutile et/ou anatase, et/ou sous une forme amorphe ou substantiellement amorphe.

Les pigments d'oxydes de titane enrobés peuvent être enrobés:
- de silice (SUNVEIL d'IKEDA) ;
- de silice et d'oxyde de fer (SUNVEIL F d'IKEDA) ;
- de silice et de polyglyceryl-10 stearate (COSMESERVE WP-40W de IWASE COSFA) ;
- de silice et d'alumine (MICROTITANIUM DIOXIDE MT500 SA et MICROTITANIUM DIOXIDE MT 100 SA de TAYCA, TIOVEIL de TIOXIDE) ;
- d'alumine (TIPAQUE TTO-55 (B) et TIPAQUE TTO-55 (A) d'ISHIHARA, et UVT 14/4 de KEMIRA) ;
- de Ti02 rutile traité avec l'alumine et de la silice enrobé de glycérol (UV TITAN M212 de KEMIRA) ;
- de TiO2 rutile traité avec l'alumine et de la diméthicone (UV TITAN M195 de KEMIRA) ;
- d'alumine et de stéarate d'aluminium (MICROTITANIUM DIOXIDE MT 100 T, MT 100 TV, MT 100 TX, MT 100 Z, MT-01 de TAYCA, Solaveil CT-10 W et Solaveil CT 100 de UNIQEMA et Eusolex T-AVO de MERCK) ;
- de silice, d'alumine et d'acide alginique (MT-100 AQ de TAYCA) ;
- d'alumine et de laurate d'aluminium (MICROTITANIUM DIOXIDE MT 100 S de TAYCA) ;
- d'alumine, de methicone et d'acide polyhydroxystearique (INP60T7 de KOBO) ;
- d'oxyde de fer et de stéarate de fer (MICROTITANIUM DIOXIDE MT 100 F de TAYCA) ;
- d'oxyde de zinc et de stéarate de zinc (BR 351 de TAYCA) ;
- de silice et d'alumine et traités par une silicone (MICROTITANIUM DIOXIDE MT 600 SAS, MICROTITANIUM DIOXIDE MT 500 SAS ou MICROTITANIUM DIOXIDE MT 100 SAS de TAYCA) ;
- de silice, d'alumine, de stéarate d'aluminium et traités par une silicone (STT-30-DS de TITAN KOGYO) ;
- d'alumine et traités par une silicone (TIPAQUE TTO-55 (S) de ISHIHARA, ou UV TITAN M 262 de KEMIRA) ;
- de triéthanolamine (STT-65-S de TITAN KOGYO) ;
- d'acide stéarique (TIPAQUE TTO-55 (C) de ISHIHARA ;
- d'hexamétaphosphate de sodium (MICROTITANIUM DIOXIDE MT 150 W de TAYCA) ;
- de TiO2 traité par l'octyl triméthyl silane (T 805 par la société DEGUSSA SILICES) ;
- de TiO2 traité par un polydiméthylsiloxane (70250 Cardre UF TiO2S13 par CARDRE) ;
- de TiO2 anatase/rutile traité par un polydiméthylhydrogénosiloxane (MICRO TITANIUM DIOXYDE USP GRADE HYDROPHOBIC par COLOR TECHNIQUES) ;
- de TiO2 rutile traité par l'alumine, l'acide stéarique (UV TITAN M160 par KEMIRA) ;
- de TiO2 traité par l'alumine hydroxyde, l'acide stéarique et le triethoxycaprylylsilane (ALT-T-400 par MAPRECOS ou Titanium Dioxide & Aluminium hydroxide & Stearic acid (ST-705SA /TITAN KOGYO)) ;
- de Ti02 dopé au manganese (OPT1-PW de Croda).

Les pigments d'oxyde de titane non enrobés sont par exemple :
- MICROTITANIUM DIOXIDE MT 500 B ou MICROTITANIUM DIOXIDE MT600 B par la société TAYCA ;
- P 25 par la société DEGUSSA ;
- Oxyde de titane transparent PW par la société WACKHER ;
- UFTR par la société MIYOSHI KASEI ;
- ITS par la société TOMEN ;
et TIOVEIL AQ par la société TIOXIDE.

Les pigments d'oxyde de zinc non enrobés, sont par exemple:
- Z-cote par la société Sunsmart;
- Nanox par la société Elementis;
- Nanogard WCD 2025 par la société Nanophase Technologies.

Les pigments d'oxyde de zinc enrobés sont par exemple:
- Oxide zinc CS-5 par la société Toshibi (ZnO enrobé par polymethylhydrogenesiloxane);
- Nanogard Zinc Oxide FN par la société Nanophase Technologies (en dispersion à 40% dans le Finsolv TN, benzoate d'alcools en C12-C15);
- DAITOPERSION ZN-30 et DAITOPERSION Zn-50 par la société Daito (dispersions dans cyclopolyméthylsiloxane /polydiméthylsiloxane oxyéthyléné, contenant 30% ou 50% de nano-oxydes de zinc enrobés par la silice et le s polyméthylhydrogènesiloxane);
- NFD Ultrafine ZnO par la société Daikin (ZnO enrobé par phosphate de perfluoroalkyle et copolymère à base de perfluoroalkyléthyle en dispersion dans du cyclopentasiloxane) ;
- SPD-Z1 par la société Shin-Etsu (ZnO enrobé par polymère acrylique greffé silicone, dispersé dans cyclodiméthylsiloxane);
- Escalol Z100 par la société ISP (ZnO traité alumine et dispersé dans le mélange methoxycinnamate d'ethylhexyle / copolymère PVP-hexadecene / methicone);
- Fuji ZnO-SMS-10 par la société Fuji Pigment (ZnO enrobé silice et polymethylsilsesquioxane);
- Nanox Gel TN par la société Elementis (ZnO dispersé à 55% dans du benzoate d'alcools en C12-C15 avec polycondensat d'acide hydroxystéarique) ;
- OTS-5 MZ-500 par la société DAITO (ZnO dispersé dans triethoxycaprylylsilane).

Les pigments d'oxyde de cérium non enrobés peuvent être par exemple ceux vendus sous la dénomination COLLOIDAL CERIUM OXIDE par la société RHONE POULENC.

### Composition - pigments

La composition contenue dans l'article selon l'invention peut comprendre de 0 à 50% en poids d'au moins un pigment.

Par "pigments", il faut comprendre des particules blanches ou colorées, minérales et/ou organiques, insolubles dans une solution aqueuse, destinées à colorer et/ou opacifier la composition et/ou le dépôt réalisé à partir de la composition.

Les pigments peuvent être choisis parmi les pigments minéraux, les laques organiques, les nacres, les pigments à effet optiques, comme les particules réfléchissantes ou les pigments interférentiels.

Les pigments minéraux peuvent être choisis parmi les pigments d'oxyde métallique, les oxydes de chrome, les oxydes de fer, le dioxyde de titane, les oxydes de zinc, les oxydes de cérium, les oxydes de zirconium, le violet de manganèse, le bleu de Prusse, le bleu outremer, le bleu ferrique, l'hydrate de chrome et leurs mélanges.

La composition contenue dans l'article selon l'invention peut également comprendre un oxyde de titane pigmentaire particulier enrobé par de l'hydroxyde d'aluminium et de l'acide stéarique commercialisé par la société Titan Kogyo sous le nom ST 705 SA. Ce pigment, déjà cité dans le paragraphe relatif aux filtres ultraviolets, présente une forte transmittance des rayons de couleur rouge tout en bloquant les autres rayons du spectre visible, et augmente le facteur de protection solaire.

La composition cosmétique peut également comprendre 0 à 50%, de préférence 0 à 30% en poids par rapport au poids total de la composition d'au moins un pigment interférentiel. Les pigments interférentiels sont des pigments aptes à générer de la couleur par un phénomène d'interférence. Ils peuvent être constitués d'un substrat, par exemple le mica naturel, la silice, le mica synthétique, le dioxyde de titane ou le verre, autour duquel sont déposées une ou plusieurs couches d'un matériau d'indice de réfraction différent comme le dioxyde de titane, l'oxyde de fer ou la silice par exemple.

A titre illustratif des pigments interférentiels pouvant être introduits dans la composition, on peut citer les pigments interférentiels de couleur or notamment commercialisés par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les pigments interférentiels bronzes notamment commercialisés par la société MERCK sous la dénomination Bronze fine (17384) (Colorona) et Bronze (17353) (Colorona) et par la société ENGELHARD sous la dénomination Super bronze (Cloisonne) ; les pigments interférentiels oranges notamment commercialisés par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les pigments interférentiels de teinte brune notamment commercialisés par la société ENGELHARD sous la dénomination Nu-antique copper 340XB (Cloisonne) et Brown CL4509 (Chromalite) ; les pigments interférentiels à reflet cuivre notamment commercialisés par la société ENGELHARD sous la dénomination Copper 340A (Timica) ; les pigments interférentiels à reflet rouge notamment commercialisés par la société MERCK sous la dénomination Sienna fine (17386) (Colorona) ; les pigments interférentiels à reflet jaune notamment commercialisés par la société ENGELHARD sous la dénomination Yellow (4502) (Chromalite) ; les pigments interférentiels de teinte rouge à reflet or notamment commercialisés par la société ENGELHARD sous la dénomination Sunstone G012 (Gemtone) ; les pigments interférentiels roses notamment commercialisés par la société ENGELHARD sous la dénomination Tan opale G005 (Gemtone) ; les pigments interférentiels noires à reflet or notamment commercialisés par la société ENGELHARD sous la dénomination Nu antique bronze 240 AB (Timica), les pigments interférentiels bleus notamment commercialisés par la société MERCK sous la dénomination Matte blue (17433) (Microna), les pigments interférentiels blancs à reflet argenté notamment commercialisés par la société MERCK sous la dénomination Xirona Silver et les pigments interférentiels orangés rosés vert doré notamment commercialisés par la société MERCK sous la dénomination Indian summer (Xirona) et leurs mélanges.

La composition peut également comprendre des laques organiques. Les laques organiques sont des pigments organiques formés d'un colorant fixé sur un substrat. Elles peuvent être par exemple choisies parmi :
- le carmin de cochenille ;
- les pigments organiques de colorants azoïques, anthraquinoniques, indigoïdes, xanthéniques, pyréniques, quinoliniques, de triphénylméthane, de fluorane. Parmi les pigments organiques, on peut notamment citer ceux connus sous les dénominations suivantes : D&C Blue n.deg. 4, D&C Brown n.deg. 1, D&C Gréen n.deg. 5, D&C Gréen n.deg. 6, D&C Orange n.deg. 4, D&C Orange n.deg. 5, D&C Orange n.deg.10, D&C Orange n.deg. 1 1, D&C Red n.deg. 6, D&C Red n.deg. 7, D&C Red n.deg. 17, D&C Red n.deg.21, D&C Red n.deg. 22, D&C Red n.deg. 27, D&C Red n.deg. 28, D&C Red n.deg. 30, D&C Red n.deg. 31, D&C Red n.deg. 33, D&C Red n.deg. 34, D&C Red n.deg. 36, D&C Violet n.deg. 2, D&C Yellow n.deg. 7, D&C Yellow n.deg. 8, D&C Yellow n.deg. 10, D&C Yellow n.deg. 1 1, FD&C Blue n.deg. 1, FD&C Gréen n.deg. 3, FD&C Red n.deg. 40, FD&C Yellow n.deg. 5, FD&C Yellow n.deg. 6.
- les sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane, ces colorants pouvant comporter au moins un groupe acide carboxylique ou sulfonique.

Les laques organiques peuvent également être supportées par un support organique tel que la colophane ou le benzoate d'aluminium, par exemple.

On peut également citer les colorants liposolubles tels que par exemple le rouge Soudan, le DC Red 17, le DC Gréen 6, le .beta.-carotène, l'huile de soja, le brun Soudan, le DC Yellow 1 1, le DC Violet 2, le DC orange 5, le jaune quinoléine.

Les matériaux chimiques correspondant à chacune des matières colorantes organiques citées précédemment sont mentionnés dans l'ouvrage " International Cosmetic Ingrédient Dictionnary and Handbook ", Edition 1997, pages 371 à 386 et 524 à 528, publié par " The Cosmetic, Toiletry, and Fragrance Association ".

### Composition - charges

La composition cosmétique peut également comporter au moins une charge, c'est-à-dire au moins une particule insoluble non colorée. Celle-ci peut être de nature minérale ou organique, de toute forme. Les charges ont pour effet de modifier la rhéologie de la composition, la texture, la sensorialité et/ou le rendu maquillage de la composition cosmétique en conférant des propriétés additionnelles telles que la matité, l'uniformité, la couvrance et/ou la stabilité.

On peut citer à titre d'exemples de charges minérales le talc, le mica, la silice, les microsphères de silice creuses, le carbonate de calcium, le carbonate de magnésium, le nitrure de bore, les microsphères de verre ou de céramique, les poudres composites de silice et de dioxyde de titane, le kaolin.

Comme exemples de charges organiques, on peut citer les poudres de polyamide, les poudres de polyéthylene, les poudres de polyméthyl méthacrylate, les poudres de polytétrafluoroéthylene (Téflon), les poudres de résine de silicone (par exemple Tospearl de Toshiba), les cires micronisées naturelles ou synthétiques, les savons métalliques dérivés d'acides carboxyliques ayant 8 à 22 atomes de carbones, les particules solides d'organopolysiloxane réticulé élastomèrique telle que celle vendue sous la référence commerciale Dow Corning EP-9215 Cosmetic Powder par Dow Corning, les particules solides d'organopolysiloxane réticulé élastomèrique enrobées de résine de silicone, en particulier enrobées d'un résine silsesquioxane telles que celles vendues sous les références commerciales KSP-101, KSP-102, KSP-103, KSP-104, KSP-105 de Shin Etsu, et leurs mélanges.

Tous les composés sous forme de particules solides peuvent avoir subi un traitement de surface, notamment afin de les rendre hydrophobes ou hydrophiles. Les pigments, les filtres minéraux, et/ou les charges peuvent être enrobés après avoir subi un ou plusieurs traitements de surface.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.

### Composition - gélifiants de la phase grasse

La composition contenue dans l'article selon l'invention peut comprendre au moins un gélifiant et/ou épaississant de la phase grasse, en plus de l'agent thixotrope contenu dans la phase aqueuse.

Parmi les gélifiants de la phase grasse utilisables dans le cadre de l'invention, on peut notamment citer les élastomères d'organopolysiloxane (également appelé élastomère de silicone) qui peuvent par exemple se présenter sous forme de particules véhiculées dans au moins une huile non volatile siliconée ou hydrocarbonée, formant ainsi un gel. Il s'agit plus particulièrement d'un élastomère de silicone réticulé. L'élastomère présent dans la composition contenue dans l'article selon l'invention peut être choisi parmi les élastomères non émulsionnants ou émulsionnants.

Par " élastomère d'organopolysiloxane émulsionnant ", on entend un élastomère d'organopolysiloxane comprenant au moins une chaîne hydrophile, tels que les élastomères d'organopolysiloxane polyoxyalkylénés (polyoxyéthylénés, polyoxypropylénés) et les élastomères de silicone polyglycérolés. Comme élastomères d'organopolysiloxane polyoxyalkylénés, on peut utiliser ceux commercialisés sous les dénominations "KSG-21", "KSG-20", "KSG-30", "KSG-31", "KSG-33", "KSG-210", "KSG-310", "KSG-330", "KSG-340" par la société Shin Etsu, "DC9010", "DC9011" par la société Dow Corning. Comme élastomères d'organopolysiloxane polyglycérolés, on peut utiliser ceux vendus sous les dénominations " KSG-710 ", " KSG-810 ", " KSG-820 ", " KSG-830 ", " KSG-840 " par la société Shin Etsu.

Comme élastomères non-émulsionnants, on peut par exemple utiliser ceux commericalisés sous les dénominations "DC 9040", "DC 9041", "DC 9509", "DC 9505" par la société Dow Corning; "KSG-6", "KSG-15", " KSG-16", " KSG-18", "KSG-41", "KSG-42", "KSG-43", "KSG-44", par la société Shin Etsu ; Gransil SR 5CYC gel, Gransil SR DMF 10 gel, Gransil SR DC556 gel de la société Gransil RPS de Grant Industries ; 1229-02-167, 1229-02-168 et " SFE 839 " de la société General Electric.

Les gélifiants de la phase grasse utilisables dans la composition contenue dans l'article selon l'invention peuvent être des argiles modifiées afin de les rendre lipophiles. On peut citer les argiles montmorillonites modifiées hydrophobes comme les bentonites ou hectorites modifiées hydrophobes. On peut citer par exemple le produit Stearalkonium Bentonite (nom INCI) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom INCI) (produit de réaction de l'hectorite et du chlorure de distéaryldimonium) vendu sous le nom de Bentone 38 ou Bentone Gel par la société Elementis Specialities.

### Composition - ingrédients cosmétiques usuels

La composition cosmétique peut également comprendre des ingrédients cosmétiques usuels additionnels, choisi notamment parmi les antioxydants, les parfums, les conservateurs, les neutralisants, les vitamines, les agents hydratant, les composés auto-bronzants, les actifs anti-rides, les actifs hydrophiles ou lipophiles, les agents anti-radicaux libres, les agents déodorants, les sequestrants, et leurs mélanges.

L'invention pourra être mieux comprise à l'aide de l'exemple de réalisation non limitatifs décrit ci-après, et à l'examen du dessin annexé sur lequel :
- la figure 1 représente en vue éclaté un exemple de réalisation de l'article cosmétique selon l'invention,
- la figure 2 représente une vue en coupe de l'article cosmétique de la figure 1,
- la figure 3 est une vue en coupe plus précise d'un détail de la figure 2, et
- la figure 4 représente un tissage en tricot préféré pour la maille de l'article cosmétique selon l'invention.

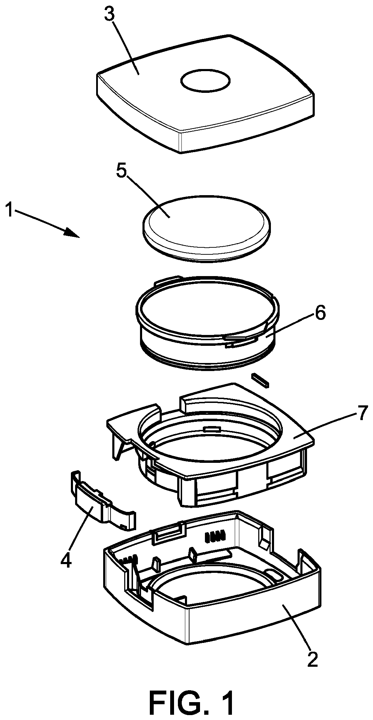

L'article cosmétique 1 illustré aux figure 1 à 3 comprend un boîtier pour produit cosmétique comprenant un corps principal dans lequel un récipient 6 rechargeable et amovible peut être mis en place.

Le boîtier est réalisé en polypropylène ou en ABS. Il est composé d'un fond 2 servant de logement pour le récipient de recharge, et d'un couvercle 3 articulé en rotation sur le fond 2, dont l'ouverture est soumise à l'actionnement d'un bouton-poussoir 4. Une houppe 5, ou une éponge applicatrice, vient se positionner entre le récipient rechargeable 6 et le couvercle 3 lorsque le boîtier est en position de fermeture

Afin d'aménager le logement, le fond est équipé d'une platine 7, dont les parois sont équipées de saillies.

Le récipient rechargeable 6 comporte un corps 61 faisant office de réservoir au produit cosmétique comportant un fond, des parois et une ouverture au sommet permettant de délivrer le produit cosmétique. Il comprend au niveau de sa base une rainure qui permet au récipient de venir s'emboîter dans les saillies de la paroi de la platine. C'est de cette manière que le récipient est mis en place et bloqué à l'intérieur du boîtier par le truchement d'un mécanisme d'encliquetage.

Le récipient comporte en outre un couvercle 62, articulé en rotation sur le corps 2 et associé à un mécanisme de fermeture permettant de maintenir le couvercle fermé contre le corps. Ce mécanisme est équipé de trois saillies, venant s'introduire dans des fentes du corps pour réaliser un encliquetage, ce qui permet d'assurer l'étanchéité du dispositif après la fermeture du couvercle sur le corps du récipient. Le couvercle peut éventuellement être équipé d'un joint 63 en Polyethylene terephthalate PET, collé sur la face intérieure couvercle 62.

Une maille 8 s'étend au-dessus de l'ouverture du corps, tendue et surmoulée à un anneau 9 venant s'encocher au corps 61 du récipient 6. Le surmoulage peut avoir été effectué par toute technique, notamment par ultrasons. L'anneau 9 est de préférence réalisé en ABS, il présente une paroi externe, une paroi interne, une surface supérieure et une surface inférieure. Il comporte une encoche sur sa paroi externe, configurée pour accueillir la saillie du corps du récipient de façon à permettre l'emboîtement du corps 61 et de l'anneau 9. Une surface plane est réalisée sur la surface supérieure, s'étendant à partir du bord intérieure de l'anneau. Cette surface peut par exemple accueillir le joint 63. La maille 8 est tendue au niveau de cette surface plane.

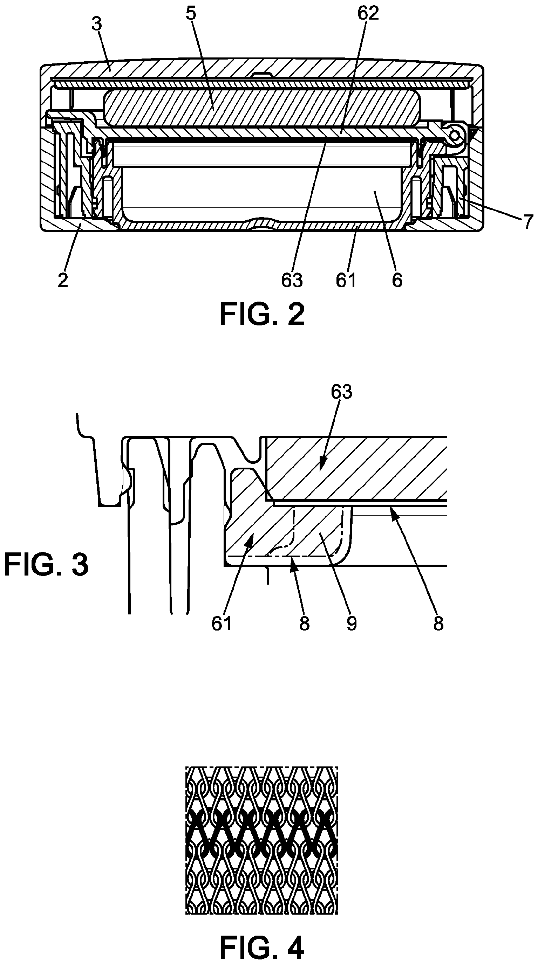

La maille 8 est configurée de façon à ce que la délivrance du produit contenu dans le corps du récipient puisse s'effectuer à travers elle. La maille est composée d'une majorité de fibres en polyuréthane, et d'une minorité de fibres en PET. Ces fibres sont tissées selon un tissage en tricot illustré à la figure 4. Cela lui confère une élasticité suffisante pour permettre d'atteindre le fond et les coins du corps du récipient.

Le réservoir contient une composition cosmétique thixotrope. Cette composition a par exemple la formulation de la composition 1 ou de la composition 2 du tableau 1 ci-dessous.

Dans le tableau 1, les quantités sont exprimées en pourcentage en poids par rapport au poids total de la composition.

**Tableau 1 : Formulations des compositions 1 à 4**

| Classe d'ingrédients / Nom INCI | Composition 1 | Composition 2 | Composition 3 | Composition 4 |
|---|---|---|---|---|
| PEG-10 DIMETHICONE | - | - | - | 6 |
| DIMETHICONE & TRIMETHYLSILOXYSILICATE/DIMETHICONOL CROSSPOLYMER | - | - | - | 2 |
| METHYL TRIMETHICONE | - | - | - | 7 |
| CYCLOPENTASILOXANE | - | - | - | 11,15 |
| DISTEARDIMONIUM HECTORITE | - | - | - | 0,15 |
| ETHYLHEXYL METHOXYCINNAMATE & BHT | 6,8 | 6,8 | - | 3 |
| TITANIUM DIOXIDE & STEARIC ACID & ALUMINA & SILICA | 3 | 3 | - | 5 |
| TRIMETHYLSILOXYSILICATE | - | - | - | 4 |
| POLYMETHYLSILSESQUIOXANE | - | - | 2 | 2 |
| DIMETHICONE/VINYL DIMETHICONE CROSSPOLYMER & ISOCETETH-10 | - | - | - | 6 |
| METHYL METHACRYLATE CROSSPOLYMER | - | - | - | 2,5 |
| CALCIUM SILICATE | - | - | - | 0,6 |
| POLYSORBATE 85 & AQUA (WATER) | 4 | 4 | - | - |
| PEG-240/HDI COPOLYMER BIS-DECYLTETRADECETH-20 ETHER & AQUA (WATER) & POTASSIUM LAURATE & TOCOPHEROL | 3,25 | 3,25 | - | - |
| SILICA | 3 | 3 | | |
| ISONONYL ISONONANOATE | 1,75 | 1,75 | - | - |
| HYDROGENATED LECITHIN | 1 | 1 | - | - |
| CETYL ETHYLHEXANOATE | 1 | 1 | | |
| DICAPRYLYL CARBONATE | 0,75 | 0,75 | - | - |
| SORBITAN LAURATE | 0,6 | 0,6 | 0,5 | - |
| Cl 77891 (TITANIUM DIOXIDE) & ALUMINUM HYDROXIDE & STEARIC ACID | 0,5 | 0,5 | - | - |
| VP/EICOSENE COPOLYMER | 0,5 | 0,5 | - | - |
| DIMETHICONE | 0,5 | 0,5 | 3,8 | 7 |
| POTASSIUM SORBATE | 0,45 | 0,45 | - | - |
| CAPRYLYL GLYCOL | 0,4 | 0,4 | - | - |
| HYDROXYETHYL ACRYLATE/SODIUM ACRYLOYLDIMETHYL TAURATE COPOLYMER & SORBITAN ISOSTEARATE & POLYSORBATE 60 | 0,3 | 0,3 | - | - |
| TOCOPHERYL ACETATE | 0,2 | 0,2 | 0,2 | - |
| XANTHAN GUM | 0,1 | 0,1 | - | - |
| CITRIC ACID | 0,07 | 0,07 | - | - |
| SODIUM HYALURONATE | 0,05 | 0,05 | - | - |
| SQUALANE | - | - | 6,5 | - |
| HYDROGENATED POLYISOBUTENE & DISTEARDIMONIUM HECTORITE & PROPYLENE CARBONATE | - | - | 6 | - |
| DIMETHICONE & DIMETHICONE/PEG-10/15 CROSSPOLYMER & DIPROPYLENE GLYCOL & TOCOPHEROL | - | - | 5,5 | - |
| DIMETHICONE & DIMETHICONE CROSSPOLYMER | - | - | 5 | - |
| PEG-9 POLYDIMETHYLSILOXYETHYL DIMETHICONE | - | - | 2,5 | - |
| SYNTHETIC FLUORPHLOGOPITE | - | - | 2 | - |
| ADENOSINE | - | - | 0,04 | - |
| GELLAN GUM | - | 0,1 | - | - |
| AQUA (WATER) | 56,08 | 55,98 | 38,51 | 21,75 |
| SODIUM CHLORIDE | - | - | 1 | 0,5 |
| AQUA (WATER) & PHENOXYETHANOL & BIOSACCHARIDE GUM-1 | 4 | 4 | 8 | 2 |
| GLYCERIN | 3 | 3 | 5 | 3 |
| ALCOHOL | 2 | 2 | 4,5 | 5 |
| PARFUM (FRAGRANCE) | 0,4 | 0,4 | 0,2 | 0,35 |
| Conservateurs | 0,8 | 0,8 | 0,75 | 0,5 |
| Pigments | 5,5 | 5,5 | 8 | 10,50 |
| TOTAL | 100 | 100 | 100 | 100 |

Les formulations 1 à 4 ont été caractérisées selon les méthodes décrites plus haut. Les résultats de ces caractérisations sont reportés dans le tableau ci-après.

**Tableau 2 : Caractérisation des compositions 1 à 4**

| **Composition n°** | **Viscosité plateau (Pa.s)** | **Seuil d'écoulement (Pa)** | **Viscosité 100s-1 (Pa.s)** | **Module complexe G^{∗} (Pa)** | **Angle de phase δ (degrés)** |
|---|---|---|---|---|---|
| **1** | **2743** | **25.3** | **1.3** | **314.8** | **8.2** |
| **2** | **3628** | **54.8** | **1.6** | **432.8** | **7.3** |
| **3** | **14770** | **55.9** | **3.5** | **663.6** | **6.7** |
| **4** | **822** | **1.8** | **1.6** | **29.3** | **6.6** |

Les compositions 1 et 2 présentent un seuil d'écoulement relativement bas, une rigidité modérée traduite par un module complexe G^{∗} intermédiaire, ainsi qu'une élasticité modérée traduite par un δ suffisamment bas. Ces propriétés confèrent à ces deux compositions les propriétés rhéologiques nécessaires à une bonne stabilité au transport dans l'article cosmétique selon l'invention, tout en facilitant l'extrusion de la composition à travers la maille et en adhérant à l'éponge applicatrice grâce à la combinaison d'un module complexe G^{∗} faible et d'un angle de phase δ faible. Par ailleurs, les viscosités à 100 s-1 des compositions 1 et 2 sont suffisamment faible pour que ces compositions s'étalent facilement sur la peau, à la manière qu'une composition fluide.

La composition 3 est une composition gélifiée qui possède une viscosité plateau très élevée, ainsi qu'un module G^{∗} et un seuil d'écoulement importants. Cela traduit une consistance importante au repos mais une élasticité insuffisante pour permettre son prélèvement à travers la maille de l'article selon l'invention avec une éponge applicatrice. De plus, la viscosité à 100 s-1 de cette composition est élevée, ce qui est synonyme de mauvais étalement de la composition 3 sur la peau.

La composition 4 présente un seuil d'écoulement faible, un module complexe G^{∗} faible, et un angle de phase δ faible. Cela traduit le fait que la composition 4 s'écoule spontanément. Par conséquent, la composition n'est pas compatible avec l'article cosmétique selon l'invention, car la composition pourrait s'écouler à travers la maille, ce qui n'est pas compatible avec un usage nomade du produit.

Il est entendu que les modes de réalisation décrits ne sont pas limitatifs et qu'il est possible d'apporter des améliorations à l'invention sans sortir du cadre de celle-ci.

A moins que le contraire ne soit précisé, le mot « ou » est équivalent à et/ou. De même, le mot « un » est équivalent à « au moins un » sauf si le contraire est précisé.

## Revendications

1. Article cosmétique (1) comprenant :
i. Un boitier,
ii. Un réservoir logé dans le boîtier, contenant une composition cosmétique thixotrope, et présentant une ouverture pour délivrer ladite composition cosmétique,
iii. Une maille (8) recouvrant ladite ouverture du réservoir, configurée pour être imperméable à la composition cosmétique lorsque celle-ci n'est pas soumise à une contrainte de cisaillement, et perméable à la composition cosmétique lorsque celle-ci est soumise à une contrainte de cisaillement.

2. Article cosmétique selon la revendication 1, dans lequel la composition cosmétique comprend une émulsion huile-dans-eau ayant une phase aqueuse et une phase grasse, la phase aqueuse comprenant un agent gélifiant thixotrope

3. Article cosmétique selon la revendication 2, dans lequel l'agent gélifiant thixotrope est un copolymère séquencé non ionique comportant au moins une portion hydrophile et une portion hydrophobe.

4. Article cosmétique selon la revendication 3, dans lequel les portions hydrophiles et les portions hydrophobes sont connectées par au moins une liaison uréthane.

5. Article cosmétique selon la revendication 3 ou 4, dans lequel le copolymère séquencé non ionique est choisi parmi les polyuréthanes et leurs dérivés.

6. Article cosmétique selon l'une quelconque des revendications 3 à 5, dans lequel ladite portion hydrophile est polyoxyéthylénée, et/ou dans laquelle ladite portion hydrophobe est hydrocarbonée.

7. Article cosmétique selon l'une quelconque des revendications 3 à 6, dans lequel le copolymère séquencé non ionique est constitué d'une chaîne de trois blocs successifs, le premier et le troisième bloc présentant des propriétés hydrophiles, et le deuxième bloc présentant des propriétés hydrophobes.

8. Article cosmétique selon l'une quelconque des revendications 2 à 7, dans lequel la teneur en poids de l'agent gélifiant thixotrope par rapport au poids total de la composition est compris entre 0,01% et 20%, de préférence entre 0,5 et 10%, encore de préférence de 1 à 5%.

9. Article cosmétique selon l'une quelconque des revendications précédentes, dans lequel la composition cosmétique présente une viscosité de plateau supérieure ou égale à 2500 Pa.s, de préférence supérieure ou égale à 2700 Pa.s, mesurée à 20°C à l'aide du rhéomètre DHR2, équipé d'un mobile Plan-Plan sablé de 40 mm de diamètre.

10. Article cosmétique selon l'une quelconque des revendications précédentes, dans lequel la composition cosmétique présente un seuil d'écoulement inférieur ou égal à 60 Pa, de préférence inférieur ou égal à 55 Pa, mesuré à 20°C à l'aide du rhéomètre DHR2, équipé d'un mobile Plan-Plan sablé de 40 mm de diamètre.

11. Article cosmétique selon l'une quelconque des revendications précédentes, dans lequel la composition cosmétique présente un module complexe G^{∗} compris entre 200 et 600 Pa, de préférence entre 300 et 500 Pa, mesuré à 20°C à l'aide du rhéomètre DHR2, équipé d'un mobile Plan-Plan sablé de 40 mm de diamètre.

12. Article cosmétique selon l'une quelconque des revendications précédentes, dans lequel la composition présente un angle de phase δ compris entre 7° et 20°, de préférence entre 7° et 10°, mesuré à 20°C à l'aide du rhéomètre DHR2, équipé d'un mobile Plan-Plan sablé de 40 mm de diamètre.

13. Article cosmétique selon l'une quelconque des revendications précédentes, la maille comprenant des fibres en polyéthylène téréphtalate et des fibres en polyuréthane.

14. Article cosmétique selon l'une quelconque des revendications précédentes, la maille étant constituée de fibres tissée en tricot.

15. Article cosmétique selon l'une quelconque des revendications précédentes comportant en outre des moyens assurant l'étanchéité du réservoir, de préférence un couvercle sur lequel est monté un joint, de préférence un joint en polyéthylène téréphtalate.

## Patentansprüche

1. Kosmetischer Artikel (1), umfassend:
i. ein Gehäuse,
ii. ein in dem Gehäuse befindliches Reservoir, das eine thixotrope kosmetische Zusammensetzung enthält und eine Öffnung zur Abgabe der kosmetischen Zusammensetzung aufweist,
iii. ein Netz (8), das die Öffnung des Reservoirs abdeckt und so konfiguriert ist, dass es für die kosmetische Zusammensetzung undurchlässig ist, wenn es nicht unter Scherspannung steht, und durchlässig für die kosmetische Zusammensetzung, wenn es unter Scherspannung steht.

2. Kosmetischer Artikel nach Anspruch 1, wobei die kosmetische Zusammensetzung eine ÖI-in-Wasser-Emulsion mit einer wässrigen Phase und einer Fettphase umfasst, wobei die wässrige Phase ein thixotropes Geliermittel umfasst.

3. Kosmetischer Artikel nach Anspruch 2, wobei das thixotrope Geliermittel ein nichtionisches Blockcopolymer mit mindestens einem hydrophilen Teil und einem hydrophoben Teil ist.

4. Kosmetischer Artikel nach Anspruch 3, wobei die hydrophilen Teile und die hydrophoben Teile durch mindestens eine Urethanbindung verbunden sind.

5. Kosmetischer Artikel nach Anspruch 3 oder 4, wobei das nichtionische Blockcopolymer aus Polyurethanen und Derivaten davon ausgewählt ist.

6. Kosmetischer Artikel nach einem der Ansprüche 3 bis 5, wobei der hydrophile Teil Polyoxyethylen ist und/oder der hydrophobe Teil Kohlenwasserstoff ist.

7. Kosmetischer Artikel nach einem der Ansprüche 3 bis 6, wobei das nichtionische Blockcopolymer aus einer Kette von drei aufeinanderfolgenden Blöcken besteht, wobei der erste und der dritte Block hydrophile Eigenschaften aufweisen und der zweite Block hydrophobe Eigenschaften aufweist.

8. Kosmetischer Artikel nach einem der Ansprüche 2 bis 7, wobei der Gewichtsanteil des thixotropen Geliermittels bezogen auf das Gesamtgewicht der Zusammensetzung zwischen 0,01 % und 20 %, vorzugsweise zwischen 0,5 und 10 %, weiter bevorzugt zwischen 1 und 5 % liegt.

9. Kosmetischer Artikel nach einem der vorhergehenden Ansprüche, wobei die kosmetische Zusammensetzung eine Plateau-Viskosität größer oder gleich 2500 Ps.s, vorzugsweise größer oder gleich 2700 Pa.s aufweist, gemessen bei 20°C mit dem Rheometer DHR2, das mit einem sandgestrahlten Plan-Plan-Mobil von 40 mm Durchmesser ausgestattet ist.

10. Kosmetischer Artikel nach einem der vorhergehenden Ansprüche, wobei die kosmetische Zusammensetzung eine Fließgrenze von höchstens 60 Pa, vorzugsweise von höchstens 55 Pa, aufweist, gemessen bei 20 °C mit dem Rheometer DHR2, das mit einem sandgestrahlten Plan-Plan-Mobil von 40 mm Durchmesser ausgestattet ist.

11. Kosmetischer Artikel nach einem der vorhergehenden Ansprüche, wobei die kosmetische Zusammensetzung einen komplexen Modul G^{∗} zwischen 200 und 600 Pa, vorzugsweise zwischen 300 und 500 Pa, aufweist, gemessen bei 20°C mit dem Rheometer DHR2, das mit einem sandgestrahlten Plan-Plan-Mobil von 40 mm Durchmesser ausgestattet ist.

12. Kosmetischer Artikel nach einem der vorhergehenden Ansprüche, wobei die Zusammensetzung einen Phasenwinkel δ zwischen 7° und 20°, vorzugsweise zwischen 7° und 10°, aufweist, gemessen bei 20°C mit dem Rheometer DHR2, das mit einem sandgestrahlten Plan-Plan-Mobil von 40 mm Durchmesser ausgestattet ist.

13. Kosmetischer Artikel nach einem der vorhergehenden Ansprüche, wobei das Netz Polyethylenterephthalat-Fasern und Polyurethan-Fasern umfasst.

14. Kosmetischer Artikel nach einem der vorhergehenden Ansprüche, wobei das Netz aus Fasern im Strickgewebe gebildet ist.

15. Kosmetischer Artikel nach einem der vorhergehenden Ansprüche, der ferner Mittel umfasst, welche die Dichtigkeit des Reservoirs gewährleisten, vorzugsweise einen Deckel, auf dem eine Dichtung, vorzugsweise eine Polyethylenterephthalat-Dichtung, angebracht ist.

## Claims

1. A cosmetic item (1) comprising:
i. A casing,
ii. A reservoir housed within the casing, containing a thixotropic cosmetic composition, and having an opening to deliver said cosmetic composition,
iii. A mesh (8) covering said opening of the reservoir, configured to be impermeable to the cosmetic composition when the latter is not subjected to a shear stress, and permeable to the cosmetic composition when the latter is subjected to a shear stress.

2. The cosmetic item according to claim 1, wherein the cosmetic composition comprises an oil-in-water emulsion having an aqueous phase and a fatty phase, the aqueous phase comprising a thixotropic gelling agent.

3. The cosmetic item according to claim 2, wherein the thixotropic gelling agent is a non-ionic block copolymer including at least one hydrophilic portion and a hydrophobic portion.

4. The cosmetic item according to claim 3, wherein the hydrophilic portions and the hydrophobic portions are connected by at least one urethane bond.

5. The cosmetic item according to claim 3 or 4, wherein the non-ionic block copolymer is selected among polyurethanes and derivatives thereof.

6. The cosmetic item according to any one of claims 3 to 5, wherein said hydrophilic portion is polyoxyethylenated and/or wherein said hydrophobic portion is hydrocarbonated.

7. The cosmetic item according to any one of claims 3 to 6, wherein the non-ionic block copolymer consists of a chain of three successive blocks, the first and third blocks having hydrophilic properties, and the second block having hydrophobic properties.

8. The cosmetic item according to any one of claims 2 to 7, wherein the weight content of the thixotropic gelling agent with respect to the total weight of the composition is comprised between 0.01% and 20%, preferably between 0.5 and 10%, still preferably from 1 to 5%.

9. The cosmetic item according to any one of the preceding claims, wherein the cosmetic compositions has a plateau viscosity higher than or equal to 2500 Pa.s, preferably higher than or equal to 2700 Pa.s, measured at 20°C using a DHR2 rheometer, equipped with a 40 mm-diameter sand-blasted parallel plate geometry.

10. The cosmetic item according to any one of the preceding claims, wherein the cosmetic composition has a flow threshold lower than or equal to 60 Pa, preferably lower than or equal to 55 Pa, measured at 20°C using a DHR2 rheometer, equipped with a 40 mm-diameter sand-blasted parallel plate geometry.

11. The cosmetic item according to any one of the preceding claims, wherein the cosmetic composition has a complex modulus G^{∗} comprised between 200 and 600 Pa, preferably between 300 and 500 Pa, measured at 20°C using a DHR2 rheometer, equipped with a 40 mm-diameter sand-blasted parallel plate geometry.

12. The cosmetic item according to any one of the preceding claims, wherein the composition has a phase angle δ comprised between 7° and 20°, preferably between 7° and 10°, measured at 20°C using a DHR2 rheometer, equipped with a 40 mm-diameter sand-blasted parallel plate geometry.

13. The cosmetic item according to any one of the preceding claims, the mesh comprising polyethylene terephthalate fibres and polyurethane fibres.

14. The cosmetic item according to any one of the preceding claims, the mesh consisting of knitted fibres.

15. The cosmetic item according to any one of the preceding claims, further including means ensuring sealing of the reservoir, preferably a cap on which a gasket is mounted, preferably a gasket made of polyethylene terephthalate.
